Europäisches Patentamt

European Patent Office

Office européen des brevets

(11) Veröffentlichungsnummer: **0 472 144 A2**

# EUROPÄISCHE PATENTANMELDUNG

(21) Anmeldenummer: **91113870.9**

(22) Anmeldetag: **19.08.91**

(51) Int. Cl.5: **C01B 33/34**, B01J 39/14, B01J 29/02, B01J 20/10, A61K 47/02, C09C 1/28

(30) Priorität: **21.08.90 DE 4026379**

(43) Veröffentlichungstag der Anmeldung:
**26.02.92 Patentblatt 92/09**

(84) Benannte Vertragsstaaten:
**CH DE ES FR GB IT LI NL SE**

(71) Anmelder: **VEREINIGTE ALUMINIUM-WERKE AKTIENGESELLSCHAFT**
**Georg-von-Boeselager-Strasse 25**
**W-5300 Bonn 1(DE)**

(72) Erfinder: **Tissler, Arno Dr.**
**Koernicke Strasse 2**
**W-5300 Bonn 1(DE)**
Erfinder: **Thome, Roland Dr.**
**Brüsseler Strasse 58**
**W-5300 Bonn 1(DE)**

(74) Vertreter: **Müller-Wolff, Thomas, Dipl.-Ing.**
**Vereinigte Aluminium-Werke AG**
**Georg-von-Boeselager-Strasse 25 Postfach 2468**
**W-5300 Bonn 1(DE)**

(54) **Verfahren zur Herstellung von Schichtsilikaten des Magadiit-Typs.**

(57) 2.1 Magadiit ist ein selten vorkommendes, natürliches Mineral, ein aluminiumfreies Natriumsilikathydrat mit Schichtstruktur der Zusammensetzung $Na_2O \cdot 14SiO_2 \cdot 9H_2O$. Die entwickelten Verfahren zur Herstellung von Magadiit weisen lange Kristallisationszeiten und damit geringe Raum-Zeit-Ausbeuten auf.

2.2 Es soll ein für den technischen Einsatz geeignetes Verfahren zur kostengünstigen, umweltschonenden Herstellung von Magadiit zur Verfügung gestellt werden. Das Verfahren ist dadurch gekennzeichnet, daß im Reaktionsansatz folgende Molverhältnisse vorliegen:

$Na_2O/SiO_2$ = 0,04 - 2
$H_2O/SiO_2$ = 2 - 100

und die hydrothermale Kristallisation in zwei Stufen erfolgt, wobei die erste Stufe den Temperaturbereich von 423 K bis 598 K bei 1 bis 20 Minuten und die zweite Stufe den Temperaturbereich von 323 K bis 423 K bei 1 - 100 Stunden umfaßt.

2.3 Die mit dem erfindungsgemäßen Verfahren hergestellten Schichtsilikate werden bevorzugt als Ionentauschmaterial, als Katalysator oder Katalysatorkomponente, als Adsorbens, als Trägermaterial für katalytisch oder pharmakologisch aktive Stoffe sowie als Füllstoff und/oder Pigment verwendet.

EP 0 472 144 A2

Die vorliegende Erfindung betrifft ein Verfahren zur Herstellung von Schichtsilikaten des Magadiit-Typs. Synthetische, kristalline Schichtsilikate dieses Typs können aufgrund ihrer chemischen, strukturellen und morphologischen Eigenschaften als Ionentauschmaterialien, als Katalysatorkomponenten, als Adsorbentien, als Trägermaterialien für katalytisch oder pharmakologisch wirksame Stoffe sowie als Füllstoffe und/oder Pigmente Verwendung finden.

Magadiit ist ein selten vorkommendes, natürliches Mineral (Science 157, (1967), 1177 ff), ein aluminiumfreies Natriumsilikathydrat mit Schichtstruktur der Zusammensetzung $Na_2O.14SiO_2.9H_2O$. Das erfindungsgemäß hergestellte Magadiit weist im Röntgendiffraktogramm zumindest die zu den in Tabelle 1 aufgeführten Netzebenenabständen zugehörigen Röntgenbeugungsreflexe auf.

Um für den technischen Einsatz dieses interessanten Materials genügend Material zur Verfügung zu stellen, sind in den letzten Jahren eine Reihe von Arbeiten unternommen worden. Die hierbei erzielten Ergebnisse bzw. die entwickelten Herstellungsverfahren weisen jedoch eine Reihe schwerwiegender Mängel, wie z.B. lange Kristallisationszeiten und damit geringe Raum-Zeit-Ausbeuten auf. Für eine großtechnische und kostengünstige Herstellung dieses Stoffes, die einen breiten Einsatz erst ermöglicht, sind diese Verfahren daher nur sehr begrenzt geeignet.

Einen Überblick über bekannte Herstellungsverfahren gibt Tabelle 2.

Aufgabe der Erfindung ist es, ein für den technischen Einsatz geeignetes Verfahren zur kostengünstigen, umweltschonenden Herstellung von Magadiit zur Verfügung zu stellen.

Diese Aufgabe wird durch das erfindungsgemäße zweistufige, vollkontinuierlich durchführbare Herstellungsverfahren gelöst.

Ein Reaktionsansatz bestehend aus einer $SiO_2$-Quelle, einer Alkali-Quelle und Wasser mit den Molverhältnissen $Na_2O/SiO_2$ = 0,04 - 2 und $H_2O/SiO_2$ = 2 - 100 wird in 10 - 120 Sekunden auf eine Temperatur von 473 k - 598 k aufgeheizt und nach einer Verweilzeit von 1 - 20 Minuten unter turbulenten Bedingungen bei dieser Temperatur in 10 - 120 Sekunden auf eine zweite Synthesestufe im Temperaturbereich von 323 k bis 423 abgekühlt. Die Verweilzeit in der zweiten Stufe beträgt 1 - 100 Stunden.

Das Produkt wird anschließend abzogen filtriert und getrocknet.

Als Einsatzstoffe können Wassergläser, Festwassergläser, Kieselsole, Kieselsäurehydrogele, feste Kieselsäureprodukte, Fällungskieselsäuren oder pyrogene Kieselsäuren, wenn nötig unter Zusatz einer Alkaliquelle verwendet werden. Die Erfindung wird in den folgenden Beispielen näher erläutert.

Tabelle 1

| Netzebenenabstände des hergestellten Magadiits | |
|---|---|
| Netzebenenabstand d [Å] | Intensität $\Sigma$ I [%] |
| 15,35 ± 0.1 | 50 |
| 7,7 ± 0.1 | 10 |
| 5,2 ± 0.1 | 20 |
| 3,54 ± 0.05 | 20 |
| 3,45 ± 0.05 | 100 |
| 3,3 ± 0.05 | 50 |
| 3,15 ± 0.05 | 70 |
| 2,6 ± 0.05 | 5 |
| 2,35 ± 0.05 | 5 |

Tabelle 2

| Herstellungsverfahren für Magadiit | | |
|---|---|---|
| Zitat | SiO$_2$-Quelle | Synthesezeit |
| DAN SSR 174 (1967) 880 | SiO$_2$-Hydrogel | 4 - 6 Wochen |
| Z.Naturf. 28b (1973) 234 | Kieselsäure | 4 Wochen |
| DD221722 (1989) | SiO$_2$-Hydrogel | 1 - 3 Tage |
| DD221723 (1985) | Na-Wasserglas | 0,5 - 5 Tage |
| DD221724 (1989) | Na-Festwasserglas | 8 - 45 Stunden |

Beispiele:

1. Natrium-Wasserglas der Dichte 1,369 kg/l und Schwefelsäure der Dichte 1,84 kg/l werden in einem statischen Mischer kontinuierlich im Volumenverhältnis 14,32/1 gemischt, wobei ein Kieselsäurehydrogel der molaren Zusammensetzung

$Na_2O/SiO_2$ = 0.28
$OH^-/SiO_2$ = 0.16
$H_2O/SiO_2$ = 7.7

entsteht.

Diese Reaktionsmischung wird kontinuierlich über einen Vorbehälter einer Kolbenpumpe zugeführt und danach in einem Rohrsystem aus drei Wärmeaustauschern innerhalb von 1 Minute auf 250 °C aufgeheizt. Nach einer Verweilzeit von 10 Minuten in einer Verweilzeitstrecke wird die Reaktionsmischung innerhalb von 1 Minute auf 185 °C abgekühlt. In nachgeschalteten Autoklaven wird die Kristallisation nach einer durchschnittlichen dortigen Verweilzeit von 2 - 3 Stunden beendet. In einem weiteren nachgeschalteten Autoklaven wird die Reaktionsmischung durch Entspannungskühlung und Wärmetausch auf ca. 60 °C abgekühlt, kontinuierlich abgezogen und filtriert. Der Filterkuchen wird gewaschen und getrocknet und besteht zu ca. 70 % aus Magadiit und enthält keine weiteren kristallinen Fremdphasen.

2. Natrium-Wasserglas der Dichte 1,365 kg/l und Schwefelsäure der Dichte 1,84 kg/l werden in einem statischen Mischer kontinuierlich im Volumenverhältnis 14,32/1 gemischt, wobei ein Kieselsäurehydrogel der molaren Zusammensetzung

$Na_2O/SiO_2$ = 0.28
$OH^-/SiO_2$ = 0.16
$H_2O/SiO_2$ = 7.7

entsteht.

Dieser Reaktionsmischung wird 10 Gew.-% Material bezogen auf den SiO$_2$-Gehalt der Reaktionsmischung aus Beispiel 1 zugegeben und anschließend wird diese kontinuierlich über einen Vorbehälter einer Kolbenpumpe zugeführt und danach in ein Rohrsystem aus drei Wärmetauschern innerhalb von 1 Minute auf 250 °C aufgeheizt. Nach einer Verweilzeit von 10 Minuten in einer Verweilzeitstrecke wird die Reaktionsmischung innerhalb von 1 Minte auf 185 °C abgekühlt. In nachgeschalteten Autoklaven wird die Kristallisation mit einer durchschnittlichen dortigen Verweilzeit von 2 - 3 Stunden beendet. In einem weiteren nachgeschalteten Autoklaven wird die Reaktionsmischung durch Entspannungskühlung und Wärmetausch auf ca. 60 °C abgekühlt, kontinuierlich abgezogen und filtriert. Der Filterkuchen wird gewaschen und getrocknet und besteht zu ca. 95 % aus Magadiit und enthält keine weiteren kristallinen Fremdphasen.

3. Natrium-Wasserglas der Dichte 1,369 kg/l und Schwefelsäure der Dichte 1,84 kg/l werden in einem statischen Mischer im Volumenverhältnis 20,58 / 1 gemischt und anschließend diese Mischung in einem zweiten statischen Mischer im Volumenverhältnis 1 / 1 mit Wasser verdünnt, wobei ein Kieselsäurehydrogel der molaren Zusammensetzung

$Na_2O/SiO_2$ = 0,28
$OH^-/SiO_2$ = 0,28
$H_2O/SiO_2$ = 17

entsteht.

Diese Reaktionsmischung wird kontinuierlich über einen Vorbehälter einer Kolbenpumpe zugeführt und danach in einem Rohrsystem aus drei Wärmetauschern innerhalb von 1 Minute auf 250 °C aufgeheizt. Nach einer Vereilzeit von 10 Minuten in einer Verweilzeitstrecke wird die Reaktionsmischung innerhalb von 1 Minute auf 185 °C abgekühlt. In nachgeschalteten Autoklven wird die Kristallisation mit einer durchschnittlichen dortigen Verweilzeit von 2 - 3 Stunden beendet. In einem weiteren nachgeschalteten Autoklaven wird die Reaktionsmischung durch Entspannungskühlung und Wärmetausch auf ca. 60 °C abgekühlt, kontinuierlich abgezogen und filtriert. Der Filterkuchen wird gewaschen und getrocknet und besteht zu ca. 60 % aus Magadiit und enthält keine weiteren kristallinen Fremdphasen.

4. Natrium-Wasserglas der Dichte 1,369 kg/l und Schwefelsäure der Dichte 1,84 kg/l werden in einem statischen Mischer im Volumenverhältnis 20,58 / 1 gemischt und anschließend diese Mischung in einem zweiten statischen Mischer im Volumenverhältnis 1 / 1 mit Wasser verdünnt, wobei ein Kieselsäurehydrogel der molaren Zusammensetzung

$Na_2O/SiO_2 = 0,28$
$OH^-/SiO_2 = 0,28$
$H_2O/SiO_2 = 17$

entsteht.

Dieser Reaktionsmischung wird 10 Gew.-% Material bezogen auf den $SiO_2$-Gehalt der Reaktionsmischung aus Beispiel 1 zugegeben und anschließend wird diese kontinuierlich über einen Vorbehälter einer Kolbenpumpe zugeführt und danach in einem Rohrsystem aus drei Wärmetauschern innerhalb von 1 Minute auf 250 °C aufgeheizt. Nach einer Verweilzeit von 10 Minunten in einer Verweilzeitstrecke wird die Reaktionsmischung innerhalb von 1 Minute auf 185 °C abgekühlt. In nachgeschalteten Autoklaven wird die Kristallisation mit einer durchschnittlichen dortigen Verweilzeit von 2 - 3 Stunden beendet. In einem weiteren nachgeschalteten Autoklaven wird die Reaktionsmischung durch Entspannungskühlung und Wärmetausch auf ca. 60 °C abgekühlt, kontinuierlich abgezogen und filtriert. Der Filterkuchen wird gewaschen und getrocknet und besteht zu ca. 90 % aus Magadiit und enthält keine weiteren kristallinen Fremdphasen.

5. Eine wäßrige Suspension mit 51 Gew.-% amorpher pyrogener Kieselsäure wird in einem statischen Mischer im Gew.-Verhältnis 1.426 / 1 mit Natriumwasserglas vermischt, wobei ein Kieselsäurehydrogel der molaren Zusammensetzung

$Na_2O/SiO_2 = 0,077$
$OH^-/SiO_2 = 0,154$
$H_2O/SiO_2 = 4,7$

entsteht.

Diese Reaktionsmischung wird kontinuierlich über einen Vorbehälter einer Kolbenpumpe zugeführt und danach in einem Rohrsystem aus drei Wärmetauschern innerhalb von 1 Minute auf 250 °C aufgeheizt. Nach einer Verweilzeit von 10 Minuten in einer Verweilzeitstrecke wird die Reaktionsmischung innerhalb von 1 Minute auf 185 °C abgekühlt. In nachgeschalteten Autoklaven wird die Kristallisation mit einer durchschnittlichen dortigen Verweilzeit von 2 - 3 Stunden beendet. In einem weiteren nachgeschalteten Autoklaven wird die Reaktionsmischung durch Entspannungskühlung und Wärmetausch auf ca. 60 °C abgekühlt, kontinuierlich abgezogen und filtriert. Der Filterkuchen wird gewaschen und getrocknet und besteht zu ca. 80 % aus Magadiit und enthält keine weiteren kristallinen Fremdphasen.

6. Eine wäßrige Suspension mit 51 Gew.-% amorpher pyrogener Kieselsäure wird in einem statischen Mischer im Gew.-Verhältnis 1.426 / 1 mit Natriumwasserglas vermischt, wobei ein Kieselsäurehydrogel der molaren Zusammensetzung

$Na_2O/SiO_2 = 0,077$
$OH^-/SiO_2 = 0,154$
$H_2O/SiO_2 = 4,7$

entsteht.

Dieser Reaktionsmischung wird 10 Gew.-% Material bezogen auf den $SiO_2$-Gehalt der Reaktionsmischung aus Beispiel 1 zugegeben und anschließend wird diese kontinuierlich über den Vorbehälter einer Kolbenpumpe zugeführt und danach in einem Rohrsystem aus drei Wärmetauschern innerhalb von 1 Minute auf 250 °C aufgeheizt. Nach einer Verweilzeit von 10 Minuten ineiner Verweilzeitstrecke wird die Reaktionsmischung innerhalb von 1 Minute auf 185 °C abgekühlt. In nachgeschalteten Autoklaven wird die Kristallisation mit einer durchschnittlichen dortigen Verweilzeit von 2 - 3 Stunden beendet. In einem weiteren nachgeschalteten Autoklaven wird die Reaktionsmischung durch Entspannungskühlung und Wärmetausch auf ca. 60 °C abgekühlt, kontinuierlich abgezogen und filtriert. Der Filterkuchen wird gewaschen und getrocknet und besteht zu ca. 95 % aus Magadiit und enthält keine weiteren kristallinen Fremdphasen.

**Patentansprüche**

1. Verfahren zur Herstellung von Schichtsilikaten des Magadiit-Typs durch hydrothermale Kristallisation aus einem Reaktionsansatz, der in wäßrig-alkalischem Medium $SiO_2$ bzw. dessen hydratisierte Derivate oder Alkali-Silikate und ggfs. Impfkeime enthält, dadurch gekennzeichnet, daß im Reaktionsansatz folgende Molverhältnisse vorliegen:

   $Na_2O/SiO_2$ = 0,04 - 2
   $H_2O/SiO_2$ = 2 - 100

   und die hydrothermale Kristallisation in zwei Stufen erfolgt, wobei die erste Stufe den Temperaturbereich von 423 K bis 598 K bei 1 bis 20 Minunten und die zweite Stufe den Temperaturbereich von 323 K bis 423 K bei 1 - 100 Stunden umfaßt.

2. Verfahren nach Anspruch 1, dadurch gekennzeichnet, daß der Reaktionsansatz innerhalb von 10 bis 120 Sekunden auf die Temperatur der ersten Stufe aufgeheizt wird.

3. Verfahren nach einem der vorhergehenden Ansprüche, dadurch gekennzeichnet, daß der Reaktionsansatz nach Beendigung der ersten Synthesestufe in 10 bis 120 Sekunden auf die Temperatur der zweiten Stufe abgekühlt wird.

4. Verfahren nach einem der vorgehenden Ansprüche, dadurch gekennzeichnet, daß dem Reaktionsansatz kristalline oder teilkristalline Stoffe oder Keimgele mit zumindest den in Tabelle 1 angegebenen Netzebenenabständen zugegeben werden.

5. Verwendung der nach einem der vorgehenden Ansprüche hergestellte Schichtsilikate als Ionentauschmaterial, als Katalysator oder Katalysatorkomponente, als Adsorbens, als Trägermaterial für katalytisch oder pharmakologisch aktive Stoffe sowie als Füllstoff und/oder Pigment.